(19) Europäisches Patentamt
European Patent Office
Office européen des brevets

(11) Veröffentlichungsnummer : **0 442 115 B1**

(12) # EUROPÄISCHE PATENTSCHRIFT

(45) Veröffentlichungstag der Patentschrift : **24.11.93 Patentblatt 93/47**

(51) Int. Cl.$^5$ : **C07C 17/12,** C07C 25/02

(21) Anmeldenummer : **90124847.6**

(22) Anmeldetag : **20.12.90**

(54) **Verfahren zur Kernchlorierung von aromatischen Kohlenwasserstoffen.**

(30) Priorität : **16.02.90 DE 4004821**

(43) Veröffentlichungstag der Anmeldung : **21.08.91 Patentblatt 91/34**

(45) Bekanntmachung des Hinweises auf die Patenterteilung : **24.11.93 Patentblatt 93/47**

(84) Benannte Vertragsstaaten : **BE CH DE FR GB IT LI**

(56) Entgegenhaltungen :
**EP-A- 0 292 824**
**EP-A- 0 340 581**
**EP-A- 0 368 063**

(73) Patentinhaber : **BAYER AG**
**D-51368 Leverkusen (DE)**

(72) Erfinder : **Mais, Franz-Josef, Dr.**
**Gustav-Poensgen-Strasse 23**
**W-4000 Düsseldorf 1 (DE)**
Erfinder : **Fiege, Helmut, Dr.**
**Walter-Flex-Strasse 23**
**W-5090 Leverkusen 1 (DE)**

EP 0 442 115 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Kernchlorierung von aromatischen Kohlenwasserstoffen, in Gegenwart von Friedel-Crafts-Katalysatoren und in Gegenwart von Co-Katalysatoren in flüssiger Phase.

Die Umsetzung von aromatischen Kohlenwasserstoffen, wie Toluol, in flüssiger Phase mit gasförmigem Chlor zu kernsubstituierten Chlorderivaten, wie Monochlortoluol, ist bekannt (Ullmanns Enzyklopädie der Technischen Chemie, 4. Aufl., Band 9, S. 499 ff.). Man führt diese Chlorierung im allgemeinen in Gegenwart von Friedel-Crafts-Katalysatoren, wie Eisen(III)-chlorid, Antimonchloriden oder Aluminiumchlorid, durch. Das erhaltene Chlorierungsprodukt ist gewöhnlich eine Mischung aus isomeren monochlorierten und polychlorierten Verbindungen. Bei Verwendung von $FeCl_3$ erhält man beispielsweise aus Toluol ein Gemisch aus Monochlortoluolen und Dichlortoluolen; in der Monochlortoluolfraktion ist das Hauptprodukt o-Chlortoluol neben p-Chlortoluol und einem geringen Anteil an m-Chlortoluol.

Da besonders p-Chloralkylbenzole, wie p-Chlortoluol, wertvolle Zwischenprodukte darstellen, hat es in der Vergangenheit nicht an Versuchen gefehlt, die Chlorierung so zu lenken, daß das Verhältnis von o- zu p-Chloralkylbenzolen erniedrigt wird, d.h., man versuchte, Bedingungen zu finden, die die Bildung von p-Chloralkylbenzolen begünstigten.

Aus US 3 226 447 ist bekannt, daß durch Zusatz von Schwefelverbindungen mit zweiwertigem Schwefel zum Friedel-Crafts-Katalysator bei der Chlorierung von Toluol ein o/p-Verhältnis von 1,2 erhalten werden kann. Nachteilig bei diesem Verfahren ist die Tatsache, daß man dieses immer noch wenig günstige Verhältnis nur bei Anwendung von Antimonsalzen als Friedel-Crafts-Katalysatoren erreicht. Nachteilig ist weiterhin, daß die erforderlichen Mengen der Katalysatorkomponenten gemäß dem dortigen Beispiel 16 sehr hoch liegen, nämlich bei 1 Gew.-%- für jeden der beiden katalytischen Zusätze. Wie das o/p-Verhältnis mit einem Wert von > 1 zeigt, entsteht hierbei immer noch mehr o- als p-Chlortoluol.

In DE-OS 1 543 020 und US 4 031 144 wird ebenfalls die Chlorierung von Toluol beispielsweise mit $FeCl_3$ und $S_2Cl_2$ beschrieben. Das erhaltene Verhältnis von o/p = 1,03-1,10 ist immer noch unbefriedigend hoch.

In US 4 031 147, US 4 069 263, US 4 069 264 und US 4 250 122 ist die Chlorierung von Toluol mit Friedel-Crafts-Katalysatoren unter Einsatz von Thianthrenen oder substituierten Thianthrenen beschrieben. Die günstigsten erreichbaren o/p-Verhältnisse liegen bei etwa 0,7, werden jedoch entweder nur durch die Verwendung von Antimonsalzen oder im Falle der Verwendung von Eisensalzen nur bei sehr niedrigen Reaktionstemperaturen von etwa 0°C erhalten. Beides ist technisch ausgesprochen ungünstig. So wird die co-katalytische Wirkung der Thianthrene beim Einsatz von Antimonsalzen durch Eisenspuren stark behindert, was in der Technik nur schwer zu realisieren ist. Zudem ist die Reaktion so stark exotherm, daß eine Abführung der Wärme bei etwa 0°C durch Sole-Kühlung sehr aufwendig wird. Ferner werden die Thianthrene unter üblichen Reaktionsbedingungen bereits von allgegenwärtigen Wasserspuren zerstört und verlieren somit ihre Wirksamkeit.

Weiterhin ist aus US 4 289 916, EP 63 384 und EP 173 222 die Chlorierung von Toluol in Gegenwart von Lewis-Säuren und Phenoxathiinen bekannt. Das nach Beispiel 1 von EP 173 222 erreichbare o/p-Verhältnis von 0,6 wird wiederum nur durch die technisch äußerst ungünstige Verwendung von Antimonchlorid und die hohe Menge von 0,29 Gew.-% an Co-Katalysator erreicht. Bei Verwendung von $FeCl_3$ anstelle von Antimonchlorid erhält man ein o/p-Verhältnis von 0,68, allerdings wiederum nur bei der technisch äußerst ungünstigen niedrigen Reaktionstemperatur von 5°C. Bei einer technisch vorteilhaften Reaktionstemperatur von 50°C steigt das o/p-Verhältnis in Gegenwart von $FeCl_3$ und dem in EP 173 222 beanspruchten Phenoxathiin-Derivat auf 0,88; dies geht aus dem Vergleichsbeispiel Nr. 50 von EP 292 824 hervor. In den genannten Schriften US 4 289 916 und EP 63 384 wird ein günstigstes o/p-Verhältnis von etwa 0,8 beschrieben. Auch hier kann das o/p-Verhältnis auf 0,65 gesenkt werden, wenn man anstelle von $FeCl_3$ Antimonchloride und eine Reaktionstemperatur von 20°C, also technisch ungünstige Bedingungen, anwendet. Auch Phenoxathiine werden in Gegenwart von Wasserspuren zerstört.

Aus EP 126 669 ist die Toluol-Chlorierung in Gegenwart von Friedel-Crafts-Katalysatoren und N-substituierten Phenothiazinen bekannt. Das o/p-Verhältnis ist mit 0,84 auch hierbei ungünstig hoch.

Aus EP 112 722, EP 154 236 und EP 248 931 ist die Chlorierung von Toluol in Gegenwart von bestimmten Zeolithen bekannt, wobei unter Zusatz von beispielsweise Halogencarbonsäurehalogeniden als Moderatoren ein o/p-Verhältnis von etwa 0,3 erreicht wird. Nachteilig an diesem Verfahren sind die erheblichen Mengen von 5 Gew.-% Zeolith und 1 Gew.-% an Moderatoren. Wie eigene Versuche zeigten, muß dieses Ergebnis mit dem erheblichen Nachteil erkauft werden, daß in den erhaltenen Gemischen sehr große Mengen (bis zu 8 Gew.-%) an Benzylchloriden auftreten. Die Bildung von Benzylchloriden stört jedoch die nachfolgende übliche destillative Aufarbeitung in ganz außerordentlichem Maße.

Aus EP 292 824 ist die Chlorierung von Alkylbenzolen mit bis zu 12 C-Atomen in der Seitenkette in Gegenwart von Friedel-Crafts-Katalysatoren und von Thiazepin-Derivaten als Co-Katalysatoren bekannt. Kennzeichnend für die Struktur der dort beanspruchten Co-Katalysatoren ist, daß von dem Stickstoffatom im heterocyclischen 7-Ring stets 3 Einfachbindungen ausgehen. Dieses N-Atom kann laut EP 292 824 nicht eine Doppelbindung eingehen. Das nach diesem Verfahren für Toluol erreichbare o/p-Verhältnis liegt im besten Fall bei 0,64 (s. Beispiel 49 von EP 292 824).

Aus DE-OS 3 815 537 und DE-OS 3 824 068 ist weiterhin ein Verfahren zur Chlorierung von Alkylbenzolen mit bis zu 12 C-Atomen in der Seitenkette in Gegenwart von Friedel-Crafts-Katalysatoren und von Thiazocin-Derivaten als Co-Katalysatoren bekannt. Auch hier ist für die wirksame Co-Katalysator-Struktur kennzeichnend, daß von dem Stickstoffatom des heterocyclischen 8-Rings nur einfache Bindungen und keine Doppelbindungen ausgehen können. Das nach diesem Verfahren für Toluol erreichbare o/p-Verhältnis liegt im besten Fall bei 0,78 (s. Beispiel 14 von DE-OS 3 815 537 bzw. DE-OS 3 824 068).

Es wurde nun ein Verfahren zur Kernchlorierung von aromatischen Kohlenwasserstoffen der Formel

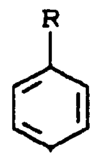

(I),

worin

R geradkettiges oder verzweigtes $C_1$-$C_{12}$-Alkyl oder $C_3$-$C_8$-Cycloalkyl bedeutet,
in Gegenwart von Friedel-Crafts-Katalysatoren und in Gegenwart von Co-Katalysatoren in flüssiger Phase gefunden, daß dadurch gekennzeichnet ist, daß man als Co-Katalysatoren am exocyclischen N-Atom oxysubstituierte cyclische Amidine der Formel

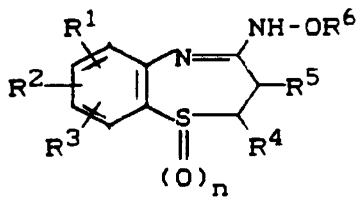

(II)

einsetzt, in der

$R^1$ und $R^2$ unabhängig voneinander Wasserstoff, Cyano, Halogen, Carboxyl, Alkoxycarbonyl, Alkyl, Aryl, Alkoxy, Aryloxy oder Acyl bedeuten,

$R^3$ für Wasserstoff, Alkyl oder Chlor steht und weiterhin mit einem der Reste $R^1$ oder $R^2$ bei benachbarter Substitution und gemeinsam mit den substituierten C-Atomen einen anellierten gesättigten, ungesättigten oder aromatischen, isocyclischen oder heterocyclischen 5-8-Ring bilden kann,

$R^4$ und $R^5$ unabhängig voneinander Wasserstoff, Alkyl, Aryl, Halogen, Alkoxy, Aryloxy, Acyl oder Acyloxy bedeuten oder gemeinsam mit den substituierten C-Atomen einen gesättigten oder ungesättigten, isocyclischen oder heterocyclischen 5-8-Ring bilden können,

$R^6$ Wasserstoff, Alkyl, Aryl oder durch Alkyl oder Aryl substituiertes Silyl bedeutet und

n den Wert null oder eins annehmen kann.

Als Halogen sei Fluor, Chlor, Brom oder Iod, bevorzugt Fluor, Chlor oder Brom, besonders bevorzugt Fluor oder Chlor genannt.

Als Alkylreste in den genannten Substituenten seien offenkettige mit 1-16 C-Atomen, bevorzugt mit 1-4 C-Atomen, und cyclische mit 5-8 C-Atomen, bevorzugt mit 5 oder 6 C-Atomen, genannt. Diese Alkylreste können ihrerseits mit $C_1$-$C_4$-Alkyl, bevorzugt mit Methyl oder Ethyl, substituiert sein, so daß man auch in die Reihe der verzweigten Alkylreste gelangt. Diese Alkylreste können weiterhin durch Fluor, Chlor oder Brom ein-oder mehrfach substituiert sein. Diese Alkylreste können weiterhin durch $C_1$-$C_4$-Alkoxy, bevorzugt durch Methoxy oder Ethoxy, substituiert sein, so daß man in die Reihe der Ether gelangt. Diese Alkylreste können weiterhin durch Phenyl, Naphthyl oder Biphenyl substituiert sein, so daß man in die Reihe der Aralkylreste gelangt. Beispiele für solche Alkylreste sind: Methyl, Ethyl, Propyl, Isopropyl, Butyl, Isobutyl, tert.-Butyl, Amyl, Hexyl, Octyl, Decyl, Dodecyl, Hexadecyl, Cyclopentyl, Methylcyclopentyl, Cyclohexyl, Methylcyclohexyl, Cycloheptyl, Cyclooctyl, Methoxymethyl, Ethoxymethyl, Benzyl, Phenylethyl, Chlormethyl, Dichlormethyl, Trichlormethyl, Trifluormethyl; besonders wichtige Reste sind beispielsweise: Methyl, Ethyl, n-Propyl, Benzyl, Trifluormethyl.

Der genannte Bedeutungsumfang für Alkylreste gilt grundsätzlich auch für Alkoxy; bevorzugt sind Reste

mit 1-6 C-Atomen, besonders bevorzugt solche mit 1-4 C-Atomen, wie Methoxy, Ethoxy, tert.-Butoxy, Cyclohexyloxy, Trifluormethoxy.

Als Arylreste in den obigen Substituenten seien beispielsweise Phenyl, Naphthyl oder Biphenyl genannt, die ihrerseits durch Fluor, Chlor, Brom, Nitro, $C_1$-$C_4$-Alkyl oder $C_1$-$C_4$-Alkoxy substituiert sein können, beispielsweise Phenyl, Naphthyl, Tolyl, Anisyl, Chlorphenyl, Nitrophenyl; besonders wichtig sind beispielsweise Phenyl und Chlorphenyl.

Bezüglich der Aryloxyreste gilt analog das oben zu den Alkoxyreste Gesagte.

Acylreste innerhalb der obigen Substituenten haben 1-8 C-Atome und sind aliphatisch, bevorzugt mit 2-4 C-Atomen, oder bei der erforderlichen Zahl von C-Atomen aromatisch. Sie können ihrerseits durch oben für Alkylreste bzw. Arylreste genannte Zweitsubstituenten substituiert sein. Als Beispiele seien genannt: Acetyl, Chloracetyl, Trichloracetyl, Trifluoracetyl, Benzoyl, Chlorbenzoyl, Chlorcarbonyl, Formyl.

Für den Fall, daß $R^3$ mit einem der Reste $R^1$ oder $R^2$ und gemeinsam mit den substituierten C-Atomen einen Ring bildet, kann dieser isocyclisch und gesättigt, ungesättigt oder aromatisch sein oder auch durch einen Gehalt an N-, O- und/oder S-Atomen heterocyclisch sein.

Solche Ringe haben 5-8, bevorzugt 5 oder 6-Ringglieder und sind an den in Formel (II) gezeigten Benzolkern anelliert. Als Beispiele für solche Ringe seien genannt: Benzo, Naphtalino, Thieno, Furano, Pyrrolo, Pyridino, Cyclohexano, Cyclopentano, Oxolano, Dioxolano, bevorzugt Benzo und Cyclohexano.

Für den Fall, daß $R^4$ und $R^5$ gemeinsam mit den substituierten C-Atomen einen Ring bilden, kann dieser isocyclisch und gesättigt oder ungesättigt oder aromatisch oder auch durch ein Gehalt an N-, O- und/oder S-Atomen heterocyclisch sein. Solche Ringe haben ebenfalls 5-8, bevorzugt 5 oder 6-Ringglieder und sind an den in der Formel (II) gezeigten Heterocyclus anelliert. Als Beispiele seien die obigen genannt, bevorzugt Cyclopentano, Cyclohexano, Dioxolano, besonders bevorzugt Cyclohexano.

Das Gerüst der erfindungsgemäßen Co-Katalysatoren wird wie folgt nummeriert:

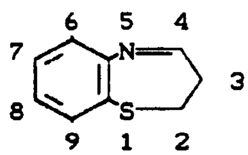

(IIa)

Da in der Formel (IIa) lediglich die Nummerierung gezeigt wird, sind zur besseren Übersichlichkeit die möglichen Substituenten und das am C-Atom in Pos. 4 sitzende oxysubstituierte N-Atom weggelassen. Die als Co-Katalysatoren erfindungsgemäß einsetzbaren Systeme der Formel (II) sind dadurch gekennzeichnet, daß bei ihnen im Gegensatz zu den Systemen von EP 292 824 zwischen dem N-Atom in Pos. 5 des heterocyclischen 7-Rings und dem benachbarten C-Atom im Pos. 4 des 7-Rings eine Doppelbindung auftritt und daß weiterhin dieses C-Atom in Pos. 4 durch ein oxysubstituiertes N-Atom, d.h. durch die Gruppierung -NH-O-$R^6$ substituiert ist. Es handelt sich somit um cyclische, am exocyclischen N-Atom oxysubstituierte Amidine, die als Derivate des Hydroxylamins aufgefaßt werden können.

Die folgende Aufzählung nennt beispielhaft solche erfindungsgemäß einsetzbaren Co-Katalysatoren, ohne die Erfindung jedoch auf diese einzuschränken:

* N-(2,3-Dihydro-1,5-benzothiazepin-4-yl)-hydroxylamin,
* N-(2-Methyl-2,3-dihydro-1,5-benzothiazepin-4-yl)-hydroxylamin,
N-(2-Ethyl-2,3-dihydro-1,5-benzothiazepin-4-yl)-hydroxylamin,
N-(2-Propyl-2,3-dihydro-1,5-benzothiazepin-4-yl)-hydroxylamin,
* N-(3-Methyl-2,3-dihydro-1,5-benzothiazepin-4-yl)-hydroxylamin,
* N-(2,3-Dimethyl-2,3-dihydro-1,5-benzothiazepin-4-yl)-hydroxylamin,
N-(2-Phenyl-2,3-dihydro-1,5-benzothiazepin-4-yl)-hydroxylamin,
N-(2-Chlor-2,3-dihydro-1,5-benzothiazepin-4-yl)-hydroxylamin,
N-(2,3-Dichlor-2,3-dihydro-1,5-benzothiazepin-4-yl)-hydroxylamin,
* N-(2-Methoxy-2,3-dihydro-1,5-benzothiazepin-4-yl)-hydroxylamin,
N-(7-Chlor-2,3-dihydro-1,5-benzothiazepin-4-yl)-hydroxylamin,
N-(6,8-Dichlor-2,3-dihydro-1,5-benzothiazepin-4-yl)-hydroxylamin,
* N-(7-Methyl-2,3-dihydro-1,5-benzothiazepin-4-yl)-hydroxylamin,
* N-(8-Methyl-2,3-dihydro-1,5-benzothiazepin-4-yl)-hydroxylamin,
* N-(7,8-Dimethyl-2,3-dihydro-1,5-benzothiazepin-4-yl)-hydroxylamin,
* N-(7,9-Dimethyl-2,3-dihydro-1,5-benzothiazepin-4-yl)-hydroxylamin,
N-(7-Trifluormethyl-2,3-dihydro-1,5-benzothiazepin-4-yl)-hydroxylamin,
* N-(8-Benzyl-2,3-dihydro-1,5-benzothiazepin-4-yl)-hydroxylamin,

N-(8-Acetyl-2,3-dihydro-1,5-benzothiazepin-4-yl)-hydroxylamin,
N-(8-Trifluoracetyl-2,3-dihydro-1,5-benzothiazepin-4-yl)-hydroxylamin,
N-(7-Phenoxy-2,3-dihydro-1,5-benzothiazepin-4-yl)-hydroxylamin,
* N-(7-Methoxy-2,3-dihydro-1,5-benzothiazepin-4-yl)-hydroxylamin,
* N-(8-Methoxy-2,3-dihydro-1,5-benzothiazepin-4-yl)-hydroxylamin,
* N-(7,8-Dimethoxy-2,3-dihydro-1,5-benzothiazepin-4-yl)-hydroxylamin,
* N-(7,9-Dimethoxy-2,3-dihydro-1,5-benzothiazepin-4-yl)-hydroxylamin,
N-(7-Phenyl-2,3-dihydro-1,5-benzothiazepin-4-yl)-hydroxylamin,
* N-(2,3-Trimethylen-2,3-dihydro-1,5-benzothiazepin-4-yl)-hydroxylamin,
* N-(2,3-Tetramethylen-2,3-dihydro-1,5-benzothiazepin-4-yl)-hydroxylamin,
* N-(7,8-Dimethyl-2,3-tetramethylen-2,3-dihydro-1,5-benzothiazepin-4-yl)-hydroxylamin,
* N-(7,9-Dimethyl-2,3-tetramethylen-2,3-dihydro-1,5-benzothiazepin-4-yl)-hydroxylamin,
* N-(7,9-Dimethyl-6-chlor-2,3-tetramethylen-2,3-dihydro-1,5-benzothiazepin-4-yl)-hydroxylamin,
* N-(2,3,7,9-Tetramethyl-2,3-dihydro-1,5-benzo-thiazepin-4-yl)-hydroxylamin,
* N-(3-Methoxy-2,3-dihydro-1,5-benzothiazepin-4-yl)-hydroxylamin,
N-(3-Acetyloxy-2,3-dihydro-1,5-benzothiazepin-4-yl)-hydroxylamin,
N-[3-Acetyloxyl-2-(methoxyphenyl)-2,3-dihydro-1,5-benzothiazepin-4-yl]-hydroxylamin,
N-(3-Acetyloxy-2-phenyl-2,3-dihydro-1,5-benzothiazepin-4-yl)-hydroxylamin,
N-(1-Oxo-2,3-dihydro-1,5-benzothiazepin-4-yl)-hydroxylamin,
N-(1-Oxo-7,9-dimethyl-2,3-tetramethylen-2,3-dihydro-1,5-benzothiazepin-4-yl)-hydroxylamin,
N-(1-Oxo-2,3,7,9-tetramethyl-2,3-dihydro-1,5-benzothiazepin-4-yl)-hydroxylamin,
* O-Methyl-N-(2,3-dihydro-1,5-benzothiazepin-4-yl)-hydroxylamin,
O-Ethyl-N-(2,3-dihydro-1,5-benzothiazepin-4-yl)-hydroxylamin,
O-Benzyl-N-(2,3-dihydro-1,5-benzothiazepin-4-yl)-hydroxylamin,
* O-Trimethylsilyl-N-(2,3-dihydro-1,5-benzothiazepin-4-yl)-hydroxylamin,
* O-Trimethylsilyl-N-(7,9-dimethyl-2,3-tetramethylen-2,3-dihydro-1,5-benzothiazepin-4-yl)-hydroxylamin,
* O-Methyl-N-(7,9-dimethyl-2,3-tetramethylen-2,3-dihydro-1,5-benzothiazepin-4-yl)-hydroxylamin;

die mit einem * vor dem Namen gekennzeichneten Verbindungen sind besonders wichtige Co-Katalysatoren.

Bevorzugte erfindungsgemäß einsetzbare Co-Katalysatoren sind allgemein am exocyclischen N-Atom oxysubstituierte cyclische Amidine der Formel

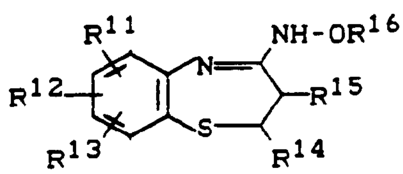

(III),

in der

| | |
|---|---|
| $R^{11}$ und $R^{12}$ | unabhängig voneinander Wasserstoff, Halogen, Alkyl oder Alkoxy bedeuten, |
| $R^{13}$ | für Wasserstoff oder Alkyl steht, |
| $R^{14}$ und $R^{15}$ | unabhängig voneinander für Wasserstoff, Chlor oder Alkyl stehen und weiterhin gemeinsam mit den substituierten C-Atomen einen gesättigten isocyclischen oder heterocyclischen 5-8-Ring bilden können und |
| $R^{16}$ | Wasserstoff, Alkyl, Aryl oder Trialkylsilyl bedeutet. |

Unter den cyclischen Amidinen der Formel (III) sind solche besonders bevorzugt, in denen an die Stelle von $R^{11}$ und $R^{12}$ die Reste $R^{22}$ und $R^{23}$ treten, die unabhängig voneinander Wasserstoff oder Alkyl bedeuten.

Unter den cyclischen Amidinen der Formel (III) sind weiterhin solche besonders bevorzugt, in denen der Rest $R^{13}$ Wasserstoff bedeutet.

Unter den cyclischen Amidinen der Formel (III) sind noch weiterhin solche besonders bevorzugt, in denen an die Stelle von $R^{14}$ und $R^{15}$ die Reste $R^{24}$ und $R^{25}$ treten, die unabhängig voneinander Wasserstoff oder Alkyl bedeuten und weiterhin gemeinsam mit den substituierten C-Atomen einen gesättigten isocyclischen 5-6-Ring bilden können.

Die erfindungsgemäß als Co-Katalysatoren einsetzbaren am exocyclischen N-Atom oxysubstituierten cyclischen Amidine können nach grundsätzlich bekannten Verfahren hergestellt werden, beispielsweise durch die Umsetzung von 4-Methylthio-2,3-dihydro-1,5-benzothiazepinen mit Hydroxylamin oder dessen Derivaten gemäß folgender Formelgleichung:

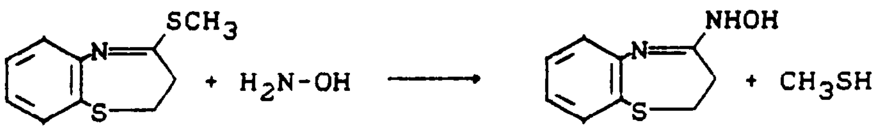

(1)

Nach J. Heterocyc. Chem. 25 (1988), 1399 gehen aus dieser Umsetzung Verbindungen des erfindungsgemäß einsetzbaren Typs hervor (vgl. Ausführungsbeispiel 49). Es ist jedoch auch möglich, solche Verbindungen durch Umsetzung der 2,3-Dihydro-1,5-benzothiazepin-5(H)-4-thione mit Hydroxylamin gemäß folgender Formelgleichung zu erhalten.

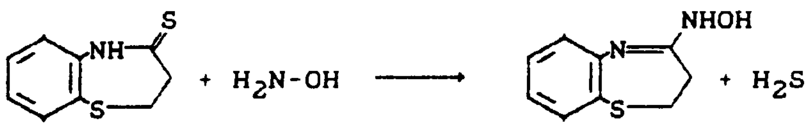

(2)

Das Produkt der Umsetzung gemäß Gleichung (2) ist identisch mit dem Produkt gemäß Gleichung (1) (vgl. Ausführungbeispiel 50).

Es ist weiterhin denkbar, daß die Verbindungen des erfindungsgemäß einsetzbaren Typs in folgendem tautomeren Gleichgewicht stehen:

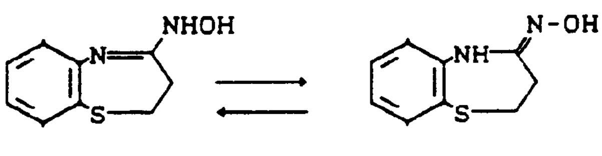

(3)

A B

Nach Aussage von J. Heterocyc. Chem. 25 (1988), 1399 liegen solche Verbindungen jedoch in der Form A vor.

Als Beispiele für die erfindungsgemäß im Kern zu chlorierenden aromatischen Kohlenwasserstoffe der Formel (I) seien genannt: Toluol, Ethylbenzol, Propylbenzol, Cumol, tert.-Butylbenzol und Phenyl-cyclohexan; das erfindungsgemäße Verfahren ist besonders wichtig für die Kernchlorierung von Toluol.

Das erfindungsgemäße Verfahren wird in flüssiger Phase durchgeführt, wobei der aromatische Kohlenwasserstoff in flüssiger (geschmolzener) Form oder gegebenenfalls in Verdünnung mit einem inerten Lösungsmittel eingesetzt werden kann. Geeignete Lösungsmittel sind solche, die durch Chlor unter den Bedingungen einer Kernchlorierung nicht angegriffen werden und dem Fachmann hierfür bekannt sind, wie Methylenchlorid, Chloroform, Tetrachlorkohlenstoff, Essigsäure. In bevorzugter Form wird ohne Lösungsmittel gearbeitet.

Als Chlorierungsmittel für das erfindungsgemäße Verfahren wird vorzugsweise Chlor verwendet. Das Chlor kann flüssig oder gasförmig in das Reaktionsgemisch eingeleitet werden. Bevorzugt wird gasförmiges Chlor eingesetzt.

Es können jedoch auch andere Chlorierungsmittel verwendet werden, die, wie beispielsweise Sulfurylchlorid, unter den Reaktionsbedingungen Chlor abgeben.

Die erfindungsgemäß durchzuführende Kernchlorierung kann grundsätzlich bei einer Temperatur vom Erstarrungspunkt bis zum Siedepunkt des Reaktionsgemisches durchgeführt werden. Im allgemeinen liegt die Reaktionstemperatur bei 0-100°C, bevorzugt 20-80°C, besonders bevorzugt 40-60°C.

Der Reaktionsdruck kann normal, vermindert oder erhöht sein und ist grundsätzlich unkritisch. Wegen der kostengünstigen Durchführung ist Normaldruck bevorzugt. Erhöhter Druck kann beispielsweise dann angezeigt sein, wenn oberhalb des Siedepunktes eines tiefsiedenden Lösungsmittels gearbeitet werden soll; in diesem Fall kann beispielsweise unter dem sich automatisch einstellenden Eigendruck des Reaktionsgemisches gearbeitet werden.

Der Chlorierungsgrad des Reaktionsgemisches liegt bevorzugt nicht wesentlich höher als 1, bezogen auf den zu chlorierenden aromatischen Kohlenwasserstoff. Höhere Chlorierungsgrade sind möglich, aber gewöhnlich nicht vorteilhaft, da sie zur Bildung unerwünschter mehrfach chlorierter Produkte führen. Chlor bzw. eine chlorabgebende Substanz wird daher beispielsweise in einer Menge von 0,8-1,1, bevorzugt 0,8-1,0 Mol pro Mol des aromatischen Kohlenwasserstoffs eingesetzt.

Der Wassergehalt der Reaktionsmischung ist im allgemeinen unkritisch. Es ist bevorzugt, alle Einsatzstoffe nicht speziell zu trocknen, sondern sie mit dem (geringen) Wassergehalt einzusetzen, mit dem sie üblicherweise in der chemischen Technik vorliegen. Es ist jedoch möglich, einzelne oder alle Stoffe des Reaktionsgemisches speziell zu trocknen. Üblicherweise sollte der Wassergehalt der Einsatzstoffe nicht über den Sättigungsgrenzen der jeweiligen Einsatzstoffe liegen. Erfindungsgemäß bevorzugt sind Wassergehalte im Chloriergemisch bis zu 250 ppm, besonders bevorzugt bis zu 150 ppm, ganz besonders bevorzugt bis zu 100 ppm.

Friedel-Crafts-Katalysatoren für das erfindungsgemäße Verfahren sind alle bekannten, beispielsweise Antimonchloride, Antiomonoxychlorid, Aluminiumchlorid, Eisen (II)-chlorid, Eisen (III)-chlorid, Tellurchloride, Molybdänchloride, Wolframchloride, Titanchloride, Zinkchlorid, Zinnchloride, Bortrichlorid und/oder Bortrifluorid. Es können jedoch auch Elemente und Elementverbindungen, die während der Chlorierung einen Friedel-Crafts-Katalysator (Lewis-Säure) bilden, eingesetzt werden, beispielsweise die elementaren Metalle oder Halbmetalle Antimon, Eisen, Blei, Zinn, Zink, Molybdän, Tellur und Aluminium oder deren Oxide, Sulfide, Carbonyle oder Salze (beispielsweise Carbonate oder ähnliche). Beispielsweise seien hierzu genannt:

Antimonoxide, Eisenoxide, Eisensulfide, Bleisulfide, Zinnsulfide, Zinksulfide, Eisencarbonyle, Molybdäncarbonyle und/oder Borphosphat. Anstelle der erwähnten Chloride können auch die Bromide, gegebenenfalls auch die Fluoride oder Iodide der genannten Elemente eingesetzt werden. Bevorzugte Friedel-Crafts-Katalysatoren sind Antimonchloride, Aluminiumchlorid, Eisen, Eisenoxide, Eisensulfide, Eisencarbonyle und/oder Eisen(III)-chlorid. Besonders bevorzugt ist Eisen(III)-chlorid.

Die Mengen des Friedel-Crafts-Katalysators oder eines Gemisches mehrerer von ihnen können in weiten Grenzen variiert werden. So ist bereits bei einem Zusatz 0,0005 Gew.-% eine Katalysatorwirkung erkennbar; andererseits können auch 5 Gew.-% oder mehr des Friedel-Crafts-Katalysators zugesetzt werden, jedoch bieten solche hohen Mengen im allgemeinen keinen Vorteil, bringen aber gegebenenfalls bei der Aufarbeitung Schwierigkeiten. Üblicherweise wird der Friedel-Crafts-Katalysator in einer Menge von 0,001-0,5 Gew-%, bevorzugt 0,01-0,1 Gew.-% eingesetzt. Alle Mengenangaben sind auf die Menge des eingesetzten aromatischen Kohlenwasserstoffs bezogen.

Die erfindungsgemäß einsetzbaren Co-Katalysatoren umfassen neben den obengenannten Substanzen alle die Substanzen, die unter den Reaktionsbedingungen Verbindungen oder Gemische von Verbindungen bilden können, die unter die Formel (II) fallen. Weiterhin einsetzbar sind alle Substanzen, die durch Reaktion der zuvorgenannten erfindungsgemäßen Co-Katalysatoren mit Chlor oder Chlorwasserstoff unter den Reaktionsbedingungen der Chlorierung gebildet werden können. Hier seien beispielsweise die Hydrochloride der obengenannten Co-Katalysatoren erwähnt.

Es ist weiterhin möglich, die Co-Katalysatoren in Kombination mit anderen, nicht als Co-Katalysatoren beanspruchten Elementen oder Verbindungen im erfindungsgemäßen Verfahren einzusetzen. Die Co-Katalysatoren können sowohl einzeln als auch im Gemisch mehrerer von ihnen eingesetzt werden. Die Mengen, in denen die erfindungsgemäßen Co-Katalysatoren eingesetzt werden, können in weiten Grenzen variieren. Mengen unter 0,0001 Gew-% sind jedoch weniger vorteilhaft, da dann die co-katalytische Wirkung nachläßt. Es können sogar Mengen von 5 Gew.-% oder mehr an Co-Katalysator zugesetzt werden, jedoch bieten diese hohen Mengen im allgemeinen keinen Vorteil, verursachen aber gegebenenfalls Aufarbeitungsprobleme. Die erfindungsgemäßen Co-Katalysatoren werden daher im allgemeinen in einer Menge von 0,0001-0,5 Gew.-%, bevorzugt 0,0005-0,1 Gew.-%, besonders bevorzugt 0,0005-0,01 Gew.-%, bezogen auf den eingesetzten aromatischen Kohlenwasserstoff, eingesetzt.

Das Molverhältnis des Gemisches aus Friedel-Crafts-Katalysator(en) und Co-Katalysator(en) kann im erfindungsgemäßen Verfahren in weiten Grenzen variiert werden. Im allgemeinen ist es vorteilhaft, den Co-Katalysator nicht in zu großem Überschuß gegenüber dem Friedel-Crafts-Katalysator einzusetzen. Ebenso ist es im allgemeinen vorteilhafter, auch den Überschuß an Friedel-Crafts-Katalysator nicht zu groß zu wählen. Erfindungsgemäß ist ein molares Verhältnis von Friedel-Crafts-Katalysator zu Co-Katalysator von 100:1-1:10, bevorzugt 75:1-1:4, besonders bevorzugt 50:1-1:2.

Für die praktische Durchführung des erfindungsgemäßen Verfahrens ist die Reihenfolge der Zugabe der einzelnen Komponenten des Reaktionsgemisches beliebig. Hierbei läßt sich das Verfahren sowohl kontinuierlich als auch diskontinuierlich durchführen. Eine beispielhafte Ausführungsform ist die folgende:

Der gewünschte aromatische Kohlenwasserstoff, beispielsweise Toluol, wird vorgelegt und auf die gewünschte Temperatur (beispielsweise 50°C) gebracht. Dann gibt man in beliebiger Reihenfolge die gewünschten Mengen an Friedel-Crafts-Katalysator(en) und Co-Katalysator(en) zu und leitet unter weitgehender Konstanthaltung der Temperatur Chlor gasförmig bis zum gewünschten Chlorierungsgrad ein. Anschließend wird das Gemisch in üblicher Weise durch Destillation aufgearbeitet.

Eine weitere beispielhafte Ausführung ist die folgende:

Man stellt eine Mischung aus Alkylbenzol mit den gewünschten Anteilen an Katalysator und Co-Katalysator her und bringt diese auf die gewünschte Reaktionstemperatur. Dann wird Chlorierungsmittel bis zum gewünschten Chlorierungsgrad eingeleitet. Die Aufarbeitung kann auch hier in üblicher Weise durch Destillation erfolgen.

Eine weitere Ausführungsform ist die folgende:

Man stellt eine Lösung von Katalysator und Co-Katalysator in dem Alkylbenzol her und führt diese einer kontinuierlich arbeitenden Chlorierapparatur zu. Man leitet, ebenfalls kontinuierlich, ein Chlorierungsmittel so schnell ein, daß der gewünschte Chlorierungsgrad erreicht wird, Auch hier kann die kontinuierlich anfallende Reaktionsmischung in üblicher Weise durch Destillation aufgearbeitet werden.

Die erfindungsgemäß geschaffene Möglichkeit der weiteren Verschiebung des Isomeren-Verhältnisses zugunsten des p-Isomeren bei der Kernchlorierung von Alkylbenzolen ist sehr überraschend, weil aufgrund der Vielzahl der in EP 292 824 bzw, in DE-OS 3 824 068 beschriebenen Thiazepine bzw. Thiazocine nicht zu erwarten war, daß ein erfindungsgemäß abgewandeltes Derivat nochmals eine solch deutliche Verbesserung, d.h. eine solch deutliche Absenkung des o/p-Verhältnisses ergibt.

Das mit dem erfindungsgemäßen Verfahren beispielsweise für die Kernchlorierung von Toluol erreichbare o/p-Verhältnis beträgt etwa 0,55. Dies ist das niedrigste o/p-Verhältnis, das bisher für Toluol mit Eisen(III)-chlorid als Friedel-Crafts-Katalysator und bei einer mittleren Temperatur von 40-60°C erreichbar ist. Eine solche Absenkung des o/p-Verhältnisses von 0,64 gemäß EP 292 824 auf etwa 0,55 bedeutet eine Steigerung des p-Anteils von zuvor 61,0 % auf nunmehr 64,5 % im Gemisch bei gleichzeitiger Abnahme des o-Anteils von bisher 39,0 % auf nunmehr 35,5 %. Das entspricht einer relativen Steigerung der p-Ausbeute von 5,7 %. Dies stellt einen erheblichen Fortschritt dar, da gemäß Aussage von EP 63 384 (dortige Seite 2, Zeile 1-6) schon Steigerungen des p-Isomerenanteils von nur 0,5 % einen hohen Fortschritt und einen großen wirtschaftlichen Wert bedeuten.

Die folgenden Beispiele sollen die Vielfalt des erfindungsgemäßen Verfahrens verdeutlichen, ohne es jedoch auf diese Beispiele einzuschränken.

## Beispiele

### Allgemeine Ausführungsvorschrift

Man legte 100 Gew.-Teile Toluol in einem Reaktor vor und gab unter Rühren die in der Tabelle angegebenen Mengen an Katalysator $FeCl_3$ und an Co-Katalysator zu. Man erhitzte auf die angegebene Temperatur und leitete unter Rühren in 5 Stunden die angegebene Chlormenge (ca. 94 Mol %) gleichmäßig ein. Das entstandene HCl-Abgas wurde über einen gut wirksamen Kühler abgeführt. Das anfallende Chloriergemisch wurde durch Gaschromatographie nach Eichung analysiert, so daß die in der Tabelle angegebene Zusammensetzung eine Zusammensetzung in Gewichtsprozent darstellt.

Das Chloriergemisch kann durch eine technisch übliche Destillation in die einzelnen Fraktionen wie Toluol, die isomeren Monochlortoluole und die Dichlortoluolfraktion zerlegt werden.

Die folgende Tabelle enthält zunächst solche erfindungsgemäßen Co-Katalysatoren, in denen $R^6$ in der Formel (II) Wasserstoff bedeutet. Dabei sind den erfindungsgemäßen Beispielen jeweils zwei Vergleichsbeispiele zugeordnet, die entweder direkt in EP 292 824 als Beispiel aufgeführt sind oder die unter den Anspruch von EP 292 824 fallen. Beispiel 1 steht also im Vergleich zu Vergleichsbeispiel 2 und Vergleichsbeispiel 3, Beispiel 4 steht im Vergleich zu den Vergleichsbeispielen 5 und 6 usw. Diese Dreiergruppen setzen sich fort bis Beispiel 34 und Vergleichsbeispiele 35 und 36.

Stellt man in den Dreiergruppen den jeweiligen erfindungsgemäßen Co-Katalysator den beiden bekannten Vergleichsbeispielen gegenüber, so ergibt sich in eindeutiger und eindrucksvoller Weise die Fähigkeit der erfindungsgemäßen Co-Katalysatoren, das o/p-Verhältnis nochmals stark abzusenken, d.h. wie gewünscht nochmals den Anteil an p-Chlortoluol im Chloriergemisch stark anzuheben.

Im weiteren schließen sich in der Tabelle die Beispiele 37 bis 40 an, die Co-Katalysatoren benutzten, in denen $R^6$ in der Formel (II) für Alkyl, Phenyl-substituiertes Alkyl oder Trialkylsilyl steht. Auch diese Beispiele wurden nach der allgemeinen Vorschrift ausgeführt.

<u>Tabelle</u>

| | Co-Katalysator Formel | Co-Katalysator Name | Temperatur °C | Co-Kat Menge in Gew.-% | $FeCl_3$ Menge in Gew.-% | eingeleit. Chlor Menge in Mol% | Zusammensetzung/Chloriergemisch | | | | | |
|---|---|---|---|---|---|---|---|---|---|---|---|---|
| | | | | | | | Toluol % | o-Cl-toluol % | m-Cl-toluol % | p-Cl-toluol % | Dichlortoluole % | Verhältnis o/p |
| Beispiel 1 | 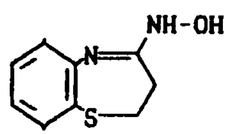 | N-(2,3-Dihydro-1,5-benzothiazepin-4-yl)-hydroxylamin | 50 | 0,0043 | 0,0175 | 94,0 | 3,79 | 37,70 | 0,65 | 57,03 | 0,83 | 0,66 |
| Vergl. beispiel 2 | 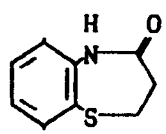 | 2,3-Dihydro-1,5-benzothiazepin-5H-4-on | 40 | 0,0040 | 0,0175 | 94,0 | 3,38 | 40,65 | 0,59 | 54,74 | 0,64 | 0,74 |
| Vergl. beispiel 3 | 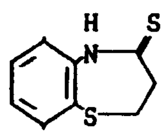 | 2,3-Dihydro-1,5-benzothiazepin-5H-4-thion | 50 | 0,0046 | 0,0175 | 94,0 | 3,36 | 41,10 | 0,69 | 54,00 | 0,85 | 0,76 |
| Beispiel 4 | 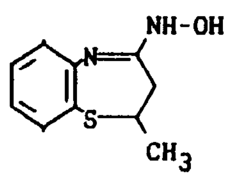 | N-(2-Methyl-2,3-dihydro-1,5-benzothiazepin-4-yl)-hydroxylamin | 50 | 0,0045 | 0,0175 | 94,0 | 2,94 | 37,88 | 0,63 | 57,61 | 0,94 | 0,66 |

**Tabelle** - Fortsetzung -

| | Co-Katalysator Formel | Co-Katalysator Name | Temperatur °C | Co-Kat Menge in Gew.-% | $FeCl_3$ Menge in Gew.-% | eingeleit. Chlor Menge in Mol% | Zusammensetzung/Chloriergemisch | | | | | |
|---|---|---|---|---|---|---|---|---|---|---|---|---|
| | | | | | | | Toluol % | o-Cl-toluol % | m-Cl toluol % | p-Cl-toluol % | Dichlortoluole % | Verhältnis o/p |
| Vergl. beispiel 5 | 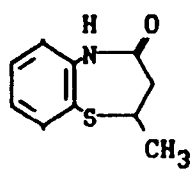 | 2-Methyl-2,3-dihydro-1,5-benzothiazepin-5H-4-on | 50 | 0,0025 | 0,0175 | 94,0 | 4,13 | 39,93 | 0,79 | 53,99 | 1,16 | 0,74 |
| Vergl. beispiel 6 | 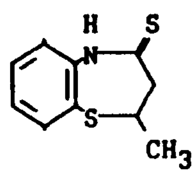 | 2-Methyl-2,3-dihydro-1,5-benzothiazepin-5H-4-thion | 50 | 0,0035 | 0,0175 | 94,8 | 2,45 | 40,84 | 0,75 | 54,23 | 1,73 | 0,75 |
| Beispiel 7 | 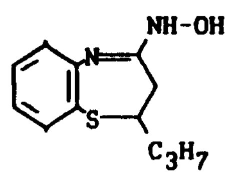 | N-(2-Propyl-2,3-dihydro-1,5-benzothiazepin-4-yl)-hydroxylamin | 50 | 0,0051 | 0,0174 | 94,0 | 4,84 | 37,81 | 0,76 | 55,28 | 1,31 | 0,68 |

**Tabelle** - Fortsetzung -

| | Co-Katalysator Formel | Co-Katalysator Name | Temperatur °C | Co-Kat Menge in Gew.-% | $FeCl_3$ Menge in Gew.-% | eingeleit. Chlor Menge in Mol% | Zusammensetzung/Chloriergemisch | | | | | |
|---|---|---|---|---|---|---|---|---|---|---|---|---|
| | | | | | | | Toluol % | o-Cl-toluol % | m-Cl toluol % | p-Cl-toluol % | Dichlor toluole % | Verhältnis o/p |
| Vergl. beispiel 8 | 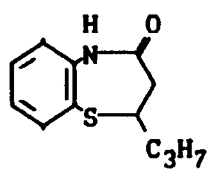 | 2-Propyl-2,3-dihydro-1,5-benzothiazepin-5H-4-on | 55 | 0,0090 | 0,0175 | 94,0 | 4,02 | 40,22 | 0,73 | 54,17 | 0,86 | 0,74 |
| Vergl. beispiel 9 | 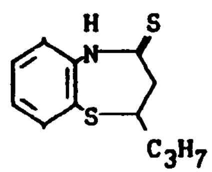 | 2-Propyl-2,3-dihydro-1,5-benzothiazepin-5H-4-thion | 50 | 0,0051 | 0,0174 | 94,0 | 4,92 | 38,86 | 0,94 | 53,93 | 1,35 | 0,72 |
| Beispiel 10 | 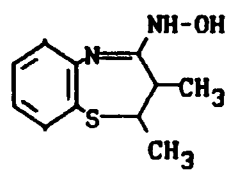 | N-(2,3-Dimethyl-2,3-dihydro-1,5-benzothiazepin-4-yl)-hydroxylamin | 55 | 0,0048 | 0,0175 | 94,0 | 3,68 | 36,68 | 0,81 | 57,63 | 1,20 | 0,64 |

<u>Tabelle</u> - Fortsetzung -

| | Co-Katalysator Formel | Co-Katalysator Name | Temperatur °C | Co-Kat Menge in Gew.-% | $FeCl_3$ Menge in Gew.-% | eingeleit. Chlor Menge in Mol% | Zusammensetzung/Chloriergemisch | | | | | |
|---|---|---|---|---|---|---|---|---|---|---|---|---|
| | | | | | | | Toluol % | o-Cl-toluol % | m-Cl toluol % | p-Cl-toluol % | Dichlortoluole % | Verhältnis o/p |
| Vergl. beispiel 11 | 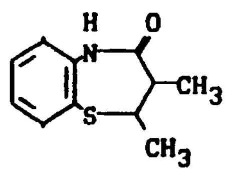 | 2,3-Dimethyl-2,3-dihydro-1,5-benzothiazepin-5H-4-on | 55 | 0,0050 | 0,0175 | 94,2 | 3,00 | 39,48 | 0,85 | 55,41 | 1,26 | 0,71 |
| Vergl. beispiel 12 | 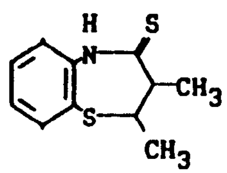 | 2,3-Dimethyl-2,3-dihydro-1,5-benzothiazepin-5H-4-thion | 55 | 0,0050 | 0,0175 | 94,0 | 3,41 | 40,09 | 0,80 | 54,52 | 1,18 | 0,73 |
| Beispiel 13 | 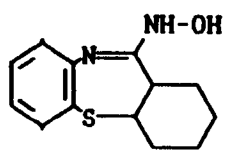 | N-(2,3-Tetramethylen-2,3-dihydro-1,5-benzothiazepin-4-yl)-hydroxylamin | 50 | 0,0053 | 0,0174 | 94,0 | 5,00 | 35,99 | 0,76 | 56,68 | 1,57 | 0,63 |

<u>Tabelle</u> - Fortsetzung -

| | Co-Katalysator Formel | Co-Katalysator Name | Temperatur °C | Co-Kat Menge in Gew.-% | $FeCl_3$ Menge in Gew.-% | eingeleit. Chlor Menge in Mol% | Zusammensetzung/Chloriergemisch | | | | | |
|---|---|---|---|---|---|---|---|---|---|---|---|---|
| | | | | | | | To-luol % | o-Cl-toluol % | m-Cl to-luol % | p-Cl-to-luol % | Di-chlor to-luole % | Ver-hält-nis o/p |
| Vergl. beispiel 14 | 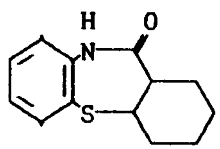 | 2,3-Tetra-methylen-2,3-di-hydro-1,5-benzo-thiazepin-5H-4-on | 50 | 0,0050 | 0,0175 | 94,0 | 4,13 | 38,29 | 0,72 | 56,08 | 0,78 | 0,68 |
| Vergl. beispiel 15 | 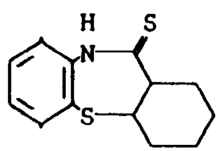 | 2,3-Tetra-methylen-2,3-dihydro-1,5-benzothiazepin-5H-4-thion | 50 | 0,0056 | 0,0174 | 94,2 | 3,13 | 38,39 | 0,80 | 56,64 | 1,04 | 0,68 |
| Beispiel 16 | 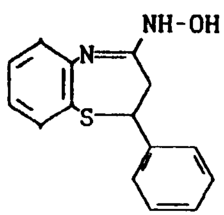 | N-(2-Phenyl-2,3-dihydro-1,5-benzothiazepin-4-yl)-hydroxyl-amin | 52 | 0,0058 | 0,0174 | 94,0 | 5,70 | 45,10 | 0,82 | 45,39 | 2,99 | 0,99 |

**Tabelle** - Fortsetzung -

| | Co-Katalysator Formel | Co-Katalysator Name | Temperatur °C | Co-Kat Menge in Gew.-% | $FeCl_3$ Menge in Gew.-% | eingeleit. Chlor Menge in Mol% | Zusammensetzung/Chloriergemisch | | | | | |
|---|---|---|---|---|---|---|---|---|---|---|---|---|
| | | | | | | | Toluol % | o-Cl-toluol % | m-Cl toluol % | p-Cl-toluol % | Dichlortoluole % | Verhältnis o/p |
| Vergl.-beispiel 17 | 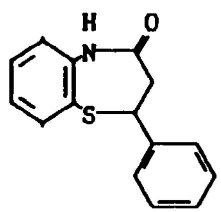 | 2-Phenyl-2,3-dihydro-1,5-benzothiazepin-5H-4-on | 55 | 0,0050 | 0,0174 | 94,0 | 7,24 | 46,93 | 1,48 | 39,34 | 5,01 | 1,19 |
| Vergl.-beispiel 18 | 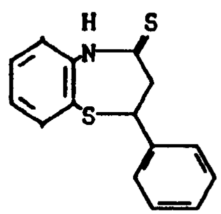 | 2-Phenyl-2,3-dihydro-1,5-benzothiazepin-5H-4-thion | 50 | 0,0061 | 0,0175 | 94,2 | 4,06 | 49,39 | 0,52 | 44,59 | 1,44 | 1,11 |
| Beispiel 19 | 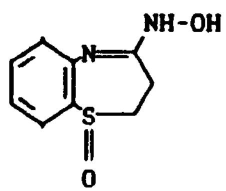 | N-(1-Oxo-2,3-dihydro-1,5-benzothiazepin-4-yl)-hydroxylamin | 50 | 0,0045 | 0,0174 | 94,0 | 4,25 | 37,90 | 0,62 | 56,77 | 0,46 | 0,67 |

**Tabelle** - Fortsetzung -

| | Co-Katalysator Formel | Co-Katalysator Name | Temperatur °C | Co-Kat Menge in Gew.-% | $FeCl_3$ Menge in Gew.-% | eingeleit. Chlor Menge in Mol% | Zusammensetzung/Chloriergemisch | | | | | |
|---|---|---|---|---|---|---|---|---|---|---|---|---|
| | | | | | | | Toluol % | o-Cl-toluol % | m-Cl toluol % | p-Cl-toluol % | Dichlortoluole % | Verhältnis o/p |
| Vergl.-beispiel 20 | 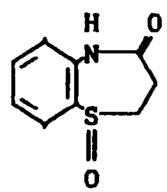 | 1-Oxo-2,3-dihydro-1,5-benzothiazepin-5H-4-on | 50 | 0,0050 | 0,0175 | 94,0 | 4,29 | 40,33 | 0,80 | 53,05 | 1,53 | 0,76 |
| Vergl.-beispiel 21 | 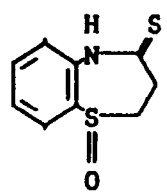 | 1-Oxo-2,3-dihydro-1,5-benzothiazepin-5H-4-thion | 50 | 0,0046 | 0,0175 | 94,0 | 4,06 | 40,78 | 0,76 | 53,51 | 0,89 | 0,76 |
| Beispiel 22 | 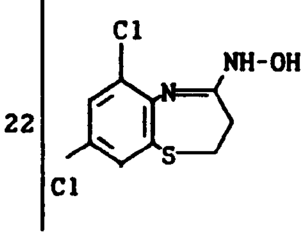 | N-(6,8-Dichlor-2,3-dihydro-1,5-benzothiazepin-4-yl)-hydroxylamin | 50 | 0,0057 | 0,0175 | 94,0 | 5,07 | 41,38 | 1,27 | 49,85 | 2,43 | 0,83 |

**Tabelle** - Fortsetzung -

| | Co-Katalysator Formel | Co-Katalysator Name | Temperatur °C | Co-Kat Menge in Gew.-% | $FeCl_3$ Menge in Gew.-% | eingeleit. Chlor Menge in Mol% | Zusammensetzung/Chloriergemisch | | | | | |
|---|---|---|---|---|---|---|---|---|---|---|---|---|
| | | | | | | | Toluol % | o-Cl-toluol % | m-Cl toluol % | p-Cl-toluol % | Dichlortoluole % | Verhältnis o/p |
| Vergl. beispiel 23 | 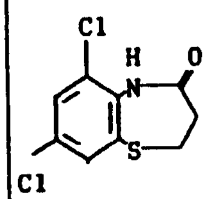 | 6,8-Dichlor-2,3-dihydro-1,5-benzothiazepin-5H-4-on | 50 | 0,0060 | 0,0175 | 94,0 | 4,37 | 44,04 | 1,11 | 48,93 | 1,55 | 0,90 |
| Vergl. beispiel 24 | 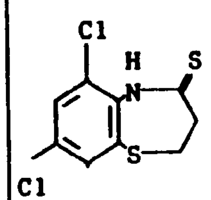 | 6,8-Dichlor-2,3-dihydro-1,5-benzothiazepin-5H-4-thion | 50 | 0,0057 | 0,0175 | 94,0 | 4,68 | 43,33 | 1,20 | 48,68 | 2,11 | 0,89 |
| Beispiel 25 | 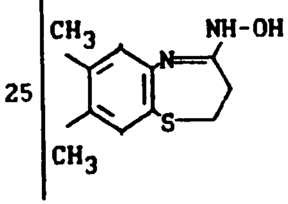 | N-(7,8-Dimethyl-2,3-dihydro-1,5-benzothiazepin-4-yl)-hydroxylamin | 50 | 0,0048 | 0,0175 | 94,0 | 3,35 | 36,06 | 0,50 | 59,12 | 0,97 | 0,61 |

**Tabelle** - Fortsetzung -

| | Co-Katalysator Formel | Co-Katalysator Name | Temperatur °C | Co-Kat Menge in Gew.-% | $FeCl_3$ Menge in Gew.-% | eingeleit. Chlor Menge in Mol% | Zusammensetzung/Chloriergemisch | | | | | |
|---|---|---|---|---|---|---|---|---|---|---|---|---|
| | | | | | | | Toluol % | o-Cl-toluol % | m-Cl toluol % | p-Cl-toluol % | Dichlortoluole % | Verhältnis o/p |
| Vergl. beispiel 26 | 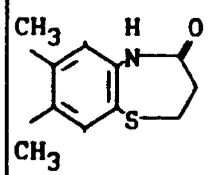 | 7,8-Dimethyl-2,3-dihydro-1,5-benzothiazepin-5H-4-on | 50 | 0,0047 | 0,0175 | 94,0 | 3,03 | 38,97 | 0,59 | 56,60 | 0,81 | 0,69 |
| Vergl. beispiel 27 | 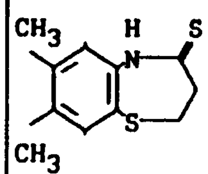 | 7,8-Dimethyl-2,3-dihydro-1,5-benzothiazepin-5H-4-thion | 50 | 0,0048 | 0,0175 | 94,0 | 3,32 | 39,63 | 0,64 | 55,83 | 0,58 | 0,71 |
| Beispiel 28 | 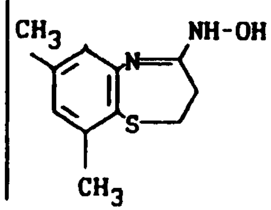 | N-(7,9-Dimethyl-2,3-dihydro-1,5-benzothiazepin-4-yl)-hydroxylamin | 50 | 0,0047 | 0,0174 | 94,0 | 4,01 | 36,15 | 0,57 | 58,05 | 1,22 | 0,62 |

Tabelle - Fortsetzung -

| | Co-Katalysator Formel | Co-Katalysator Name | Temperatur °C | Co-Kat Menge in Gew.-% | $FeCl_3$ Menge in Gew.-% | eingeleit. Chlor Menge in Mol% | Zusammensetzung/Chloriergemisch | | | | | |
|---|---|---|---|---|---|---|---|---|---|---|---|---|
| | | | | | | | Toluol % | o-Cl-toluol % | m-Cl toluol % | p-Cl-toluol % | Dichlortoluole % | Verhältnis o/p |
| Vergl. beispiel 29 | 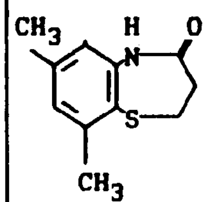 | 7,9-Dimethyl-2,3-dihydro-1,5-benzothiazepin-5H-4-on | 50 | 0,0047 | 0,0175 | 94,0 | 2,84 | 39,87 | 0,61 | 56,04 | 0,64 | 0,71 |
| Vergl. beispiel 30 | 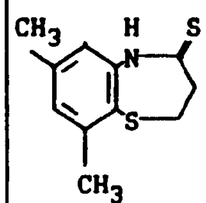 | 7,9-Dimethyl-2,3-dihydro-1,5-benzothiazepin-5H-4-thion | 50 | 0,0047 | 0,0175 | 94,0 | 3,75 | 38,46 | 0,70 | 55,46 | 1,63 | 0,69 |
| Beispiel 31 | 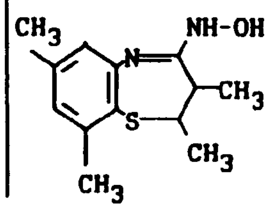 | N-(2,3,7,9-Tetramethyl-2,3-dihydro-1,5-benzothiazepin-4-yl)-hydroxylamin | 50 | 0,0054 | 0,0175 | 94,2 | 3,59 | 35,66 | 0,65 | 59,12 | 0,98 | 0,60 |

**Tabelle** - Fortsetzung -

| | Co-Katalysator Formel | Co-Katalysator Name | Temperatur °C | Co-Kat Menge in Gew.-% | $FeCl_3$ Menge in Gew.-% | eingeleit. Chlor Menge in Mol% | Zusammensetzung/Chloriergemisch | | | | | |
|---|---|---|---|---|---|---|---|---|---|---|---|---|
| | | | | | | | Toluol % | o-Cl-toluol % | m-Cl toluol % | p-Cl-toluol % | Dichlortoluole % | Verhältnis o/p |
| Vergl. beispiel 32 | 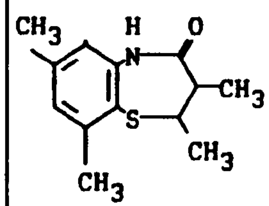 | 2,3,7,9-Tetramethyl-2,3-dihydro-1,5-benzothiazepin-5H-4-on | 55 | 0,0053 | 0,0175 | 94,0 | 3,14 | 38,29 | 0,68 | 57,10 | 0,79 | 0,67 |
| Vergl. beispiel 33 | 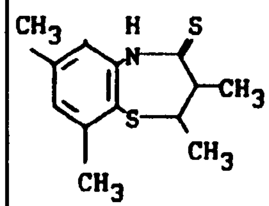 | 2,3,7,9-Tetramethyl-2,3-dihydro-1,5-benzothiazepin-5H-4-thion | 50 | 0,0054 | 0,0175 | 94,0 | 4,77 | 38,06 | 0,90 | 54,25 | 2,02 | 0,70 |
| Beispiel 34 | 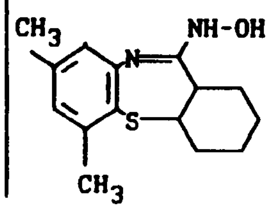 | N-(7,9-Dimethyl-2,3-tetramethylen-2,3-dihydro-1,5-benzothiazepin-4-yl)-hydroxylamin | 50 | 0,0059 | 0,0175 | 94,0 | 4,00 | 33,61 | 0,56 | 61,02 | 0,81 | 0,55 |

Tabelle - Fortsetzung -

| | Co-Katalysator Formel | Co-Katalysator Name | Temperatur °C | Co-Kat Menge in Gew.-% | $FeCl_3$ Menge in Gew.-% | eingeleit. Chlor Menge in Mol% | Zusammensetzung/Chloriergemisch: Toluol % | o-Cl-toluol % | m-Cl toluol % | p-Cl-toluol % | Dichlortoluole % | Verhältnis o/p |
|---|---|---|---|---|---|---|---|---|---|---|---|---|
| Vergl. beispiel 35 | 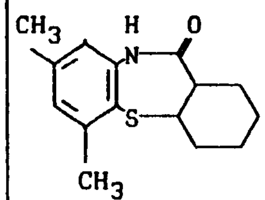 | 7,9-Dimethyl-2,3-tetramethylen-2,3-dihydro-1,5-benzothiazepin-5H-4-on | 55 | 0,0057 | 0,0175 | 94,0 | 3,37 | 37,33 | 0,65 | 57,89 | 0,76 | 0,64 |
| Vergl. beispiel 36 | 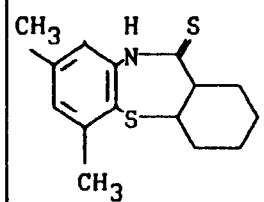 | 7,9-Dimethyl-2,3-tetramethylen-2,3-dihydro-1,5-benzothiazepin-5H-4-thion | 50 | 0,0059 | 0,0175 | 94,0 | 3,81 | 37,18 | 0,77 | 57,41 | 0,83 | 0,65 |
| Beispiel 37 | 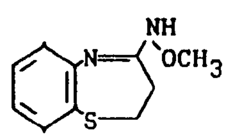 | O-Methyl-N-(2,3-dihydro-1,5-benzothiazepin-4-yl)-hydroxylamin | 50 | 0,0045 | 0,0175 | 94,0 | 3,52 | 38,08 | 0,67 | 56,37 | 1,36 | 0,67 |

**Tabelle** - Fortsetzung -

| | Co-Katalysator Formel | Co-Katalysator Name | Temperatur °C | Co-Kat Menge in Gew.-% | $FeCl_3$ Menge in Gew.-% | eingeleit. Chlor Menge in Mol% | Zusammensetzung/Chloriergemisch | | | | | |
|---|---|---|---|---|---|---|---|---|---|---|---|---|
| | | | | | | | Toluol % | o-Cl-toluol % | m-Cl toluol % | p-Cl-toluol % | Dichlortoluole % | Verhältnis o/p |
| Beisp. 38 | 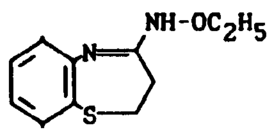 | O-Ethyl-N-(2,3-dihydro-1,5-benzothiazepin-4-yl)-hydroxylamin | 50 | 0,048 | 0,0175 | 94,0 | 4,59 | 37,69 | 0,61 | 56,24 | 0,87 | 0,67 |
| Beisp. 39 | 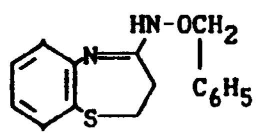 | O-Benzyl-N-(2,3-dihydro-1,5-benzothiazepin-4-yl)-hydroxylamin | 50 | 0,061 | 0,0175 | 94,0 | 4,53 | 38,14 | 0,63 | 55,74 | 0,96 | 0,68 |
| Beisp. 40 | 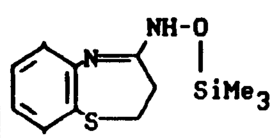 | O-Trimethylsilyl-N-(2,3-dihydro-1,5-benzothiazepin-4-yl)-hydroxylamin | 50 | 0,0072 | 0,00174 | 94,0 | 3,53 | 38,11 | 0,61 | 56,97 | 0,88 | 0,67 |

Die folgenden Beispiele und Vergleichsbeispiele erläutern die Erfindung für die Kernchlorierung von weiteren Alkylbenzolen gemäß Formel (I).

Beispiel 41

(Erfindungsgemäße Kernchlorierung von Ethylbenzol)

Es wurden 100 Gew.-Teile Ethylbenzol bei Raumtemperatur mit 0,0175 Gew.-Teilen $FeCl_3$ und 0,045 Gew.-Teilen des Co-Katalysators der Formel

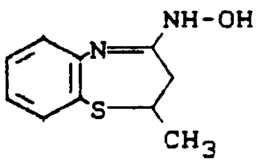

[N-(2-Methyl-2,3-dihydro-1,5-benzothiazepin-4-yl)-hydroxylamin]

versetzt und auf 50°C erhitzt. Analog der allgemeinen Vorschrift wurden 94,0 Mol% $Cl_2$ in 5 h gleichmäßig eingeleitet. Das Chlorierungsgemisch wurde durch Gaschromatographie analysiert. Die Zusammensetzung des Chloriergemisches war wie folgt: 6,75 Gew.-% Ethylbenzol, 29,60 Gew.-% ortho-Chlorethylbenzol, 0,71 Gew.-% meta-Chlorethylbenzol, 62,21 Gew.-% para-Chlorethylbenzol und 0,73 Gew.-% Dichlorethylbenzole. Das o/p-Verhältnis beträgt somit 0,47.

Vergleichsbeispiel 42

(Kernchlorierung von Ethylbenzol gemäß EP 292 824)

Das Verfahren von Beispiel 41 wurde wiederholt, wobei jedoch statt des dortigen Co-Katalysators 0,0050 Gew.-Teile des Co-Katalysators der Formel

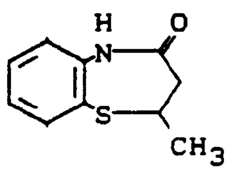

[2-Methyl-2,3-dihydro-1,5-benzothiazepin-5H-4-on]

zugegeben wurden. Die Zusammensetzung des Chloriergemisches war wie folgt:
4,22 Gew.-% Ethylbenzol, 33,12 Gew.-% ortho-Chlorethylbenzol, 0,89 Gew.-% meta-Chlorethylbenzol, 60,76 Gew.-% para-Chlorethylbenzol und 1,01 Gew.-% Dichlorethylbenzole. Das o/p-Verhältnis beträgt somit 0,54.

Beispiel 43

(Erfindungsgemäße Kernchlorierung von Isopropylbenzol)

Das Verfahren von Beispiel 41 wurde wiederholt, wobei jedoch statt Ethylbenzol 100 Gew.-Teile Isopropylbenzol und statt des dortigen Co-Katalysators 0,048 Gew.-Teile des Co-Katalysators der Formel

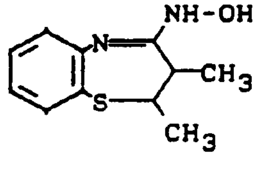

[N-(2,3-Dimethyl-2,3-dihydro-1,5-benzothiazepin-4-yl)-hydroxylamin]

angewandt wurden. Die Zusammensetzung des Chloriergemisches war wie folgt: 6,73 Gew.-% Isopropylbenzol, 18,37 Gew.-% ortho-Chlorisopropylbenzol, 1,09 Gew.-% meta-Chlorisopropylbenzol, 73,40 Gew.-% para-Chlorisopropylbenzol und 0,41 Gew.-% Dichlorisopropylbenzole. Das o/p-Verhältnis beträgt somit 0,25.

Vergleichsbeispiel 44

(Kernchlorierung von Isopropylbenzol gemäß EP 292 284)

Das Verfahren von Beispiel 41 wurde wiederholt, wobei jedoch statt Ethylbenzol 100 Gew.-Teile Isopropylbenzol und statt des dortigen Co-Katalysators 0,005 Gew.-Teile des Co-Katalysators der Formel

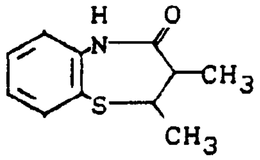

[2,3-Dimethyl-2,3-dihydro-1,5-benzothiazepin-5H-4-on]

angewandt wurden. Die Zusammensetzung des Chloriergemisches war wie folgt: 3,38 Gew.-% Isopropylbenzol, 21,17 Gew.-% ortho-Chlorisopropylbenzol, 1,38 Gew.-% meta-Chlorisopropylbenzol, 73,02 Gew.-% para-Chlorisopropylbenzol und 1,04 Gew.-% Dichlorisopropylbenzole. Das o/p-Verhältnis beträgt somit 0,29.

Beispiel 45

(Erfindungsgemäße Kernchlorierung von t.-Butylbenzol)

Das Verfahren von Beispiel 41 wurde wiederholt, wobei jedoch statt Ethylbenzol 100 Gew.-Teile t.-Butylbenzol und statt des dortigen Co-Katalysators 0,0042 Gew.-Teile des Co-Katalysators der Formel

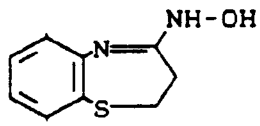

[N-(2,3-Dihydro-1,5-benzothiazepin-4-yl)hydroxylamin]

angewandt wurden. Die Zusammensetzung des Chloriergemisches war wie folgt: 7,61 Gew.-% t.-Butylbenzol, 10,63 Gew.-% ortho-Chlort.-butylbenzol, 0,89 Gew.-% meta-Chlor-t.-butylbenzol, 79,68 Gew.-% para-Chlor-t.-butylbenzol und 1,19 Gew.-% Dichlor-t.-butylbenzole. Das o/p-Verhältnis beträgt somit 0,13.

Vergleichsbeispiel 46

(Kernchlorierung von t.-Butylbenzol gemäß EP 292 824)

Das Verfahren von Beispiel 41 wurde wiederholt, wobei jedoch statt Ethylbenzol 100 Gew.-Teile t.-Butylbenzol und statt des dortigen Co-Katalysators 0,0045 Gew.-Teile des Co-Katalysators der Formel

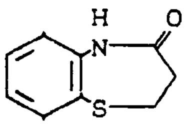

[2,3-Dihydro-1,5-benzothiazepin-5H-4-on]

angewandt wurden. Die Zusammensetzung des Chloriergemisches war wie folgt:
8,91 Gew.-% t.-Butylbenzol, 12,91 Gew.-% ortho-Chlor-t.-butylbenzol, 0,95 Gew.-% meta-Chlor-t.-butylbenzol, 75,94 Gew.-% para-Chlor-t.-butylbenzol und 1,29 Gew.-% Dichlor-t.-butylbenzole. Das o/p-Verhältnis beträgt somit 0,17.

Beispiel 47

(Erfindungsgemäße Kernchlorierung von Chlorhexylbenzol)

Das Verfahren von Beispiel 41 wurde wiederholt, wobei jedoch statt Ethylbenzol 100 Gew.-Teile Cyclohexylbenzol und statt des dortigen Co-Katalysators 0,0046 Gew.-Teile des Co-Katalysators der Formel

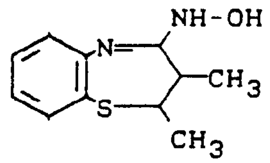

[N-(2,3-Dimethyl-2,3-dihydro-1,5-benzothiazepin-4-yl)-hydroxylamin

angewandt wurden. Die Zusammensetzung des Chloriergemisches war wie folgt:
5,09 Gew.-% Cyclohexylbenzol, 15,51 Gew.-% ortho-Chlor-cyclohexylbenzol, 0,70 Gew.-% meta-Chlorcyclohexylbenzol, 76,76 Gew.-% para-Chlor-cyclohexylbenzol und 1,94 Gew.-% Dichlor-cyclohexylbenzole. Das o/p-Verhältnis beträgt somit 0,20.

Vergleichsbeispiel 48

(Kernchlorierung von Cyclohexylbenzol gemäß EP 292 824)

Das Verfahren von Beispiel 41 wurde wiederholt, wobei jedoch statt Ethylbenzol 100 Gew.-Teile Cyclohexylbenzol und statt des dortigen Co-Katalysators 0,0045 Gew.-Teile des Co-Katalysators der Formel

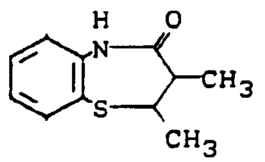

[2,3-Dimethyl-2,3-dihydro-1,5-benzothiazepin 5H-4-on]

angewandt wurden. Die Zusammensetzung des Chloriergemisches war wie folgt:
2,63 Gew.-% Cyclohexylbenzol, 19,06 Gew.-% ortho-Chlor-cyclohexylbenzol, 1,09 Gew.-% meta-Chlor-cyclohexylbenzol, 76,24 Gew.-% para-Chlor-cyclohexylbenzol und 0,98 Gew.-% Dichlor-cyclohexylbenzole. Das o/p-Verhältnis beträgt somit 0,25.

Die Beispiele und Vergleichsbeispiele 41 bis 48 zeigen eindeutig, daß auch für die Kernchlorierung von weiteren Alkylbenzolen der Formel (I) die erfindungsgemäßen Co-Katalysatoren einen niedrigen o/p-Wert (d.h. einen höheren Anteil an dem p-Chloralkylbenzolisomeren) ergeben als die z.B. aus EP 292 284 bekannten Co-Katalysatoren.

Im folgenden ist die Herstellung von Co-Katalysatoren der Formel (II) am Beispiel der Verbindung N-(2,3-Dihydro-1,5-benzothiazepin-4-yl)hydroxylamin beschrieben.

Beispiel 49

(Nach J. Heterocycl. Chem 25, (1988) 1399)

In eine Lösung von 5,25 g (25,12 mmol) 4-Methylthio-2,3-dihydro-1,5-benzothiazepin in 20 ml abs. Ethanol wurden 2,40 g (34,53 mmol) Hydroxylammoniumchlorid und 3,50 g (34,65 mmol) Triethylamin zugegeben und 20 h unter Rückfluß (~78°C) gerührt. Anschließend wurde bis zur Trockene eingedampft und der Rückstand aus 70 %igem Ethanol (Rest: $H_2O$) umkristallisiert und gut getrocknet.
Ausbeute: 2,85 g (58,5 % der theoret. Ausbeute)
Schmelzpunkt: 194-196°C (Lit. 194-195°C)
Das Produkt zeigte auf der Dünnschichtchromatographieplatte (Kieselgel)/Laufmittel: Dichlormethan nur einen Fleck.

Beispiel 50

In eine Lösung von 19,50 g (100,00 mmol) 2,3-Dihydro-1,5-benzothiazepin-5H-4-thion in 200 ml abs. Ethanol wurden 13,90 g (200,00 mmol) Hydroxylammoniumchlorid und 20,20 g (200,00 mmol) Triethylamin gegeben. Man erhitzte 6 h zum Rückfluß und dampfte dann zur Trockene ein. Der Rückstand wurde mit 0,5 l Wasser + 0,5 l Methylenchlorid ausgerührt, die Methylenchloridphase abgetrennt, mit $MgSO_4$ getrocknet und

erneut eingedampft. Dieser zweite Rückstand wurde aus 70 %igem Ethanol (Rest $H_2O$) umkristallisiert und gut getrocknet.
Ausbeute: 16,50 g (85,0 % der theoret. Ausbeute)
Schmelzpunkt: 194-196°C
Laut Schmelzpunkt und Mischschmelzpunkt sowie der DC-Vergleichsanalyse war das Produkt mit dem des Beispiels 49 identisch.

## Patentansprüche

1. Verfahren zur Kernchlorierung von aromatischen Kohlenwasserstoffen der Formel

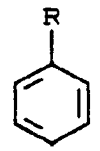

,

worin

R geradkettiges oder verzweigtes $C_1$-$C_{12}$-Alkyl oder $C_3$-$C_8$-Cycloalkyl bedeutet, in Gegenwart von Friedel-Crafts-Katalysatoren und in Gegenwart von Co-Katalysatoren in flüssiger Phase, dadurch gekennzeichnet, daß man als Co-Katalysatoren am exocyclischen N-Atom oxysubstituierte cyclische Amidine der Formel

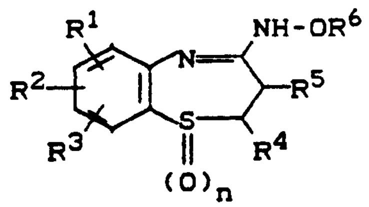

einsetzt, in der

$R^1$ und $R^2$ unabhängig voneinander Wasserstoff, Cyano, Halogen, Carboxyl, Alkoxycarbonyl, Alkyl, Aryl, Alkoxy, Aryloxy oder Acyl bedeuten,

$R^3$ für Wasserstoff, Alkyl oder Chlor steht und weiterhin mit einem der Reste $R^1$ oder $R^2$ bei benachbarter Substitution und gemeinsam mit den substituierten C-Atomen einen anellierten gesättigten, ungesättigten oder aromatischen, isocyclischen oder heterocyclischen 5-8-Ring bilden kann,

$R^4$ und $R^5$ unabhängig voneinander Wasserstoff, Alkyl, Aryl, Halogen, Alkoxy, Aryloxy, Acyl oder Acyloxy bedeuten oder gemeinsam mit den substituierten C-Atomen einen gesättigten oder ungesättigten, isocyclischen oder heterocyclischen 5-8-Ring bilden können,

$R^6$ Wasserstoff, Alkyl, Aryl oder durch Alkyl oder Aryl substituiertes Silyl bedeutet und

n den Wert null oder eins annehmen kann.

2. Verfahren nach Anspruch 1 dadurch gekennzeichnet, daß man cyclische Amidine der Formel

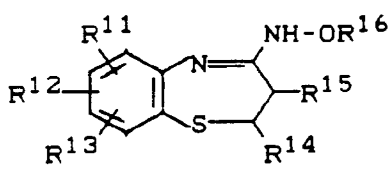

einsetzt,
in der

$R^{11}$ und $R^{12}$ unabhängig voneinander Wasserstoff, Halogen, Alkyl oder Alkoxy bedeuten,

| | |
|---|---|
| $R^{13}$ | für Wasserstoff oder Alkyl steht, |
| $R^{14}$ und $R^{15}$ | unabhängig voneinander für Wasserstoff, Chlor oder Alkyl stehen und weiterhin gemeinsam mit den substituierten C-Atomen einen gesättigten isocyclischen oder heterocyclischen 5-8-Ring bilden können und |
| $R^{16}$ | Wasserstoff, Alkyl, Aryl oder Trialkylsilyl bedeutet. |

**3.** Verfahren nach Anspruch 2 dadurch gekennzeichnet, daß an die Stelle von $R^{11}$ und $R^{12}$ die Reste $R^{22}$ und $R^{23}$ treten, die unabhängig voneinander Wasserstoff oder Alkyl bedeuten.

**4.** Verfahren nach Anspruch 2 dadurch gekennzeichnet, daß der Rest $R^{13}$ Wasserstoff bedeutet.

**5.** Verfahren nach Anspruch 2 dadurch gekennzeichnet, daß an die Stelle von $R^{14}$ und $R^{15}$ die Reste $R^{24}$ und $R^{25}$ treten, die unabhängig voneinander Wasserstoff oder Alkyl bedeuten und weiterhin gemeinsam mit den substituierten C-Atomen einen gesättigten isocyclischen 5-6-Ring bilden können.

**6.** Verfahren nach Anspruch 1 dadurch gekennzeichnet, daß die Kernchlorierung bei einer Temperatur vom Erstarrungspunkt bis zum Siedepunkt des Reaktionsgemisches, bevorzugt von 0-100°C, besonders bevorzugt von 20-80°C, ganz besonders bevorzugt von 40-60°C durchgeführt wird.

**7.** Verfahren nach Anspruch 1 dadurch gekennzeichnet, daß 0,8-1,1, bevorzugt 0,8-1,0 Mol Chlor bzw. Chlor abgebende Substanz pro Mol des aromatischen Kohlenwasserstoffes eingesetzt werden.

**8.** Verfahren nach Anspruch 1 dadurch gekennzeichnet, daß die Menge an eingesetztem Friedel-Crafts-Katalysator 0,001-0,5 Gew.-%, bevorzugt 0,01-0,1 Gew.-%, bezogen auf den eingesetzten aromatischen Kohlenwasserstoff, beträgt.

**9.** Verfahren nach Anspruch 1 dadurch gekennzeichnet, daß die Menge an eingesetztem Co-Katalysator 0,0001-0,5 Gew.-%, bevorzugt 0,0005-0,1 Gew.-%, besonders bevorzugt 0,0005-0,01 Gew.-%, bezogen auf den eingesetzten aromatischen Kohlenwasserstoff, beträgt.

**10.** Verfahren nach Anspruch 1 dadurch gekennzeichnt, daß das molare Verhältnis von Friedel-Crafts-Katalysator zu Co-Katalysator 100:1-1:10, bevorzugt 75:1-1:4, besonders bevorzugt 50:1-1:2, beträgt.

## Claims

**1.** Process for the ring-chlorination of aromatic hydrocarbons of the formula

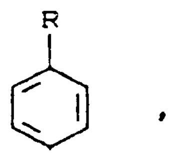

wherein

R denotes straight-chain or branched $C_1$-$C_{12}$-alkyl or $C_3$-$C_8$-cycloalkyl,

in the liquid phase in the presence of Friedel-Crafts catalysts and in the presence of co-catalysts, characterised in that the co-catalysts employed are cyclic amidines which are oxy-substituted on the exocyclic N atom, of the formula

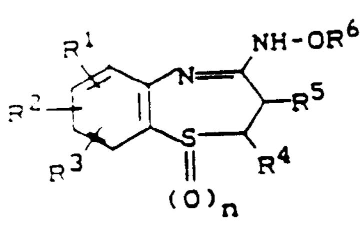

in which

| | |
|---|---|
| $R^1$ and $R^2$ | independently of one another denote hydrogen, cyano, halogen, carboxyl, alkoxycarbonyl, alkyl, aryl, alkoxy, aryloxy or acyl, |
| $R^3$ | represents hydrogen, alkyl or chlorine, and furthermore can form a fused-on saturated, unsaturated or aromatic isocyclic or heterocyclic 5- to 8-membered ring with one of the radicals $R^1$ or $R^2$ in adjacent substitution and together with the substituted C atoms, |
| $R^4$ and $R^5$ | independently of one another denote hydrogen, Alkyl, aryl, halogen,. alkoxy, aryloxy, acyl or acyloxy, or can form a saturated or unsaturated isocyclic or heterocyclic 5- to 8-membered ring together with the substituted C atoms, |
| $R^6$ | Denotes hydrogen, alkyl, aryl or silyl which is substituted by alkyl or aryl and |
| n | can assume the value zero or one. |

2. Process according to Claim 1, characterised in that cyclic amidines of the formula

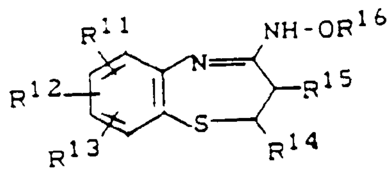

in which

| | |
|---|---|
| $R^{11}$ and $R^{12}$ | independently of one another denote hydrogen, halogen, alkyl or alkoxy, |
| $R^{13}$ | represents hydrogen or alkyl, |
| $R^{14}$ and $R^{15}$ | independently of one another represent hydrogen, chlorine or alkyl, and furthermore can for a saturated isocyclic or heterocyclic 5- to 8-membered ring together with the substituted C atoms and |
| $R^{16}$ | denotes hydrogen, alkyl, aryl or trialkylsilyl, are employed. |

3. Process according to Claim 2, characterised in that the radicals $R^{22}$ and $R^{23}$, which independently of one another denote hydrogen or alkyl, occur instead of $R^{11}$ and $R^{12}$.

4. Process according to Claim 2, characterised in that the radical $R^{13}$ denotes hydrogen.

5. Process according to Claim 2, characterised in that the radicals $R^{24}$ and $R^{25}$, which independently of one another denote hydrogen or alkyl and furthermore can form a saturated isocyclic 5- or 6-membered ring together with the substituted C atoms, occur instead of $R^{14}$ and $R^{15}$.

6. Process according to Claim 1, characterised in that the ring-chlorination is carried out at a temperature from the solidification point up to the boiling point of the reaction mixture, preferably from 0-100°C, particularly preferably 20-80°C and especially preferably 40-60°C.

7. Process according to Claim 1, characterised in that 0.8-1.1, preferably 0.8-1.0 mol of chlorine or chlorine-donating substance are employed per mol of the aromatic hydrocarbon.

8. Process according to Claim 1, characterised in that the amount of Friedel-Crafts catalyst employed is 0.001-0.5% by weight, preferably 0.01-0.1% by weight, based on the aromatic hydrocarbon employed.

9. Process according to Claim 1, characterised in that the amount of co-catalyst employed is 0.0001-0.5% by weight, preferably 0.0005-0.1% by weight, particularly preferably 0.0005-0.01% by weight, based on the aromatic hydrocarbon employed.

10. Process according to Claim 1, characterised in that the molar ratio of Friedel-Crafts catalyst to co-catalyst is 100:1-1:10, preferably 75:1-1:4, particularly preferably 50:1-1:2.

## Revendications

1. Procédé de chloration sur le noyau d'hydrocarbures aromatiques de formule

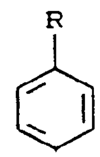

dans laquelle

R est un groupe alkyle en $C_1$ à $C_{12}$ linéaire ou ramifié ou un groupe cycloalkyle en $C_3$ à $C_8$, en présence de catalyseurs de Friedel-Crafts et en présence de cocatalyseurs en phase liquide, caractérisé en ce qu'on utilise comme cocataly-seurs des amidines cycliques à substituant oxy sur l'atome d'azote exocyclique, de formule

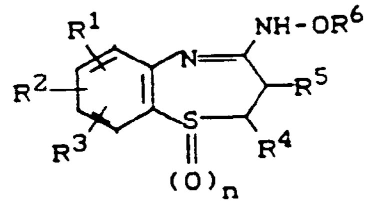

dans laquelle

$R^1$ et $R^2$ représentent, indépendamment l'un de l'autre, l'hydrogène, un groupe cyano, un halogène, un groupe carboxyle, alkoxycarbonyle, alkyle, aryle, alkoxy, aryloxy ou acyle,

$R^3$ est l'hydrogène, un groupe alkyle ou le chlore et peut former en outre conjointement avec l'un des restes $R^1$ ou $R^2$ en cas de substitution contiguë et, le cas échéant, avec les atomes substitués de carbone, un noyau pentagonal à octogonal isocyclique ou hétérocyclique, saturé, non saturé ou aromatique condensé,

$R^4$ et $R^5$ représentent, indépendamment l'un de l'autre, l'hydrogène, un groupe alkyle, aryle, un halogène, un groupe alkoxy, aryloxy, acyle ou acyloxy ou peuvent former conjointement avec les atomes substitués de carbone, un noyau pentagonal à octogonal saturé ou non saturé, isocyclique ou hétérocyclique,

$R^6$ représente l'hydrogène, un groupe alkyle, aryle ou un groupe silyle substitué par un radical alkyle ou aryle et

n peut prendre la valeur zéro ou un.

**2.** Procédé suivant la revendication 1, caractérisé en ce qu'on utilise des amidines cycliques de formule

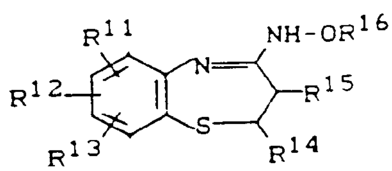

dans laquelle

$R^{11}$ et $R^{12}$ représentent, indépendamment l'un de l'autre, l'hydrogène, un halogène, un groupe alkyle ou alkoxy,

$R^{13}$ est l'hydrogène ou un groupe alkyle,

$R^{14}$ et $R^{15}$ représentent, indépendamment l'un de l'autre, l'hydrogène, le chlore ou un groupe alkyle et peuvent former en outre, conjointement avec les atomes substitués de carbone, un noyau pentagonal à octogonal isocyclique ou hétérocyclique saturé et

$R^{16}$ représente l'hydrogène, un groupe alkyle, aryle ou trialkylsilyle.

**3.** Procédé suivant la revendication 2, caractérisé en ce que les restes $R^{11}$ et $R^{12}$ sont remplacés par les restes $R^{22}$ et $R^{23}$ qui désignent, indépendamment l'un de l'autre l'hydrogène ou des groupes alkyle.

**4.** Procédé suivant la revendication 2, caractérisé en ce que le reste $R^{13}$ représente l'hydrogène.

5. Procédé suivant la revendication 2, caractérisé en ce que les restes $R^{14}$ et $R^{15}$ sont remplacés par les restes $R^{24}$ et $R^{25}$ qui représentent, indépendamment l'un de l'autre, l'hydrogène ou des groupes alkyle et peuvent en outre former, conjointement avec les atomes substitués de carbone, un noyau pentagonal ou hexagonal isocyclique saturé.

6. Procédé suivant la revendication 1, caractérisé en ce que la chloration sur le noyau est conduite à une température allant du point de solidification au point d'ébullition du mélange réactionnel, de préférence de 0 à 100°C, mieux encore de 20 à 80°C et notamment de 40 à 60°C.

7. Procédé suivant la revendication 1, caractérisé en ce qu'on utilise, par mole d'hydrocarbure aromatique, 0,8 à 1,1, de préférence 0,8 à 1,0 mole de chlore ou de substance cédant du chlore.

8. Procédé suivant la revendication 1, caractérisé en ce que la quantité utilisée de catalyseur de Friedel-Crafts s'élève à 0,001-0,5 % en poids, de préférence à 0,01-0,1 % en poids par rapport à l'hydrocarbure aromatique utilisé.

9. Procédé suivant la revendication 1, caractérisé en ce que la quantité utilisée de cocatalyseur s'élève à 0,0001-0,5 % en poids, de préférence à 0,0005-0,1 % en poids, notamment à 0,0005-0,01 % en poids par rapport à l'hydrocarbure aromatique utilisé.

10. Procédé suivant la revendication 1, caractérisé en ce que le rapport molaire du catalyseur de Friedel-Crafts au cocatalyseur s'élève à 100:1-1:10, de préférence à 75:1-1:4, notamment à 50:1-1:2.